# EUROPEAN PATENT SPECIFICATION

(11) **EP 2 126 133 B1**
(45) Date of publication and mention of the grant of the patent: **06.05.2015**
(21) Application number: 08732386.1
(22) Date of filing: 18.03.2008
(51) Int. Cl.: C12Q 1/68, C12N 15/10

(54) **MULTIPLY-PRIMED AMPLIFICATION OF CIRCULAR NUCLEIC ACID SEQUENCES**
MULTIPLY-PRIMED-AMPLIFIKATION ZIRKULÄRER NUKEINSÄURESEQUENZEN
AMPLIFICATION MULTI-AMORCEE DE SEQUENCES D'ACIDES NUCLEIQUES CIRCULAIRES

(30) Priority: 23.03.2007 US 896509 P
(43) Date of publication of application: 02.12.2009
(73) Proprietor: GE Healthcare Bio-Sciences Corp., Piscataway, N.J. 08855 (US)
(72) Inventor: XIAO, Haiguang, Princeton New Jersey 08540 (US); NELSON, John, R., Clifton Park New York 12065 (US); CHERNAYA, Galina, Princeton New Jersey 08540 (US); WANG, Haiying, Bridgewater New Jersey 08807 (US); MITSIS, Paul, Trenton New Jersey 08618 (US); KUMAR, Gyanendra, Somerset New Jersey 08873 (US); FULLER, Carl, Berkeley Heights New Jersey 07922 (US); CAI, Yuyang, Christine, Cranbury New Jersey 08512 (US)
(74) Representative: Bannan, Sally
(86) International application number: PCT/US2008/057301
(87) International publication number: WO 2008/118679

(56) References cited:
- EP-A- 1 895 013
- WO-A-02/38755
- WO-A-2006/003721
- US-A1- 2005 019 776
- US-B1- 6 323 009
- FREDRIKSSON SIMON ET AL: "Multiplex amplification of all coding sequences within 10 cancer genes by Gene-Collector." NUCLEIC ACIDS RESEARCH 2007, vol. 35, no. 7, February 2007 (2007-02), page e47, XP002487873 ISSN: 1362-4962
- NELSON J R ET AL: "TEMPLIPHI, PHI29 DNA POLYMERASE BASED ROLLING CIRCLE AMPLIFICATION OF TEMPLATES FOR DNA SEQUENCING" BIOTECHNIQUES, INFORMA LIFE SCIENCES PUBLISHING, WESTBOROUGH, MA, US, 1 June 2002 (2002-06-01), pages S44-S47, XP001204520 ISSN: 0736-6205
- DEAN F B ET AL: "Rapid amplification of plasmid and phage DNA using Phi29 DNA polymerase and multiply-primed rolling circle amplification" GENOME RESEARCH, COLD SPRING HARBOR LABORATORY PRESS, WOODBURY, NY, US, vol. 11, no. 6, 1 June 2001 (2001-06-01), pages 1095-1099, XP002223174 ISSN: 1088-9051
- GAUBATZ J W ET AL: "PURIFICATION OF EUCARYOTIC EXTRACHROMOSOMAL CIRCULAR DNAS USING EXONUCLEASE III" 19900101, vol. 184, no. 2, 1 January 1990 (1990-01-01), pages 305-310, XP001024672
- HYMAN, EDWARD DAVID: 'Preparation of plasmid DNA by sequential enzymatic digestion' BIOTECHNIQUES vol. 13, no. 4, 1992, pages 550, 552 - 554, XP008165156 ISSN: 0736-6205

### SEQUENCE LISTING

<110> GE HEALTHCARE BIO-SCIENCES CORP.
   XIAO, Haiguang
   NELSON, John
   CHERNAYA, Galina
   WANG, Haiying
   MITSIS, Paul
   KUMAR, Gyanendra
   FULLER, Carl
   CAI, Yuyang Christine
<120> MULTIPLY-PRIMED AMPLIFICATION OF CIRCULAR NUCLEIC ACID SEQUENCES
<130> PB0733
<150> US 60/896,509
   <151> 2007-03-23
<160> 6
<170> PatentIn version 3.3
<210> 1
   <211> 21
   <212> DNA
   <213> Artificial Sequence
<220>
   <223> Synthetic Primer
<400> 1
   ttgacgttac ccgcagaaga a 21
<210> 2
   <211> 20
   <212> DNA
   <213> Artificial Sequence
<220>
   <223> Synthetic Primer
<400> 2
   tgcgctttac gcccagtaat 20
<210> 3
   <211> 21
   <212> DNA
   <213> Artificial Sequence
<220>
   <223> Synthetic Primer
<400> 3
   tccctaccga ttatgagttt g 21
<210> 4
   <211> 20
   <212> DNA
   <213> Artificial Sequence
<220>
   <223> Synthetic Primer
<400> 4
   cccttggcct aggatacaag 20
<210> 5
   <211> 20
   <212> DNA
   <213> Artificial Sequence
<220>
   <223> Synthetic Primer
<400> 5
   cgatttaggt gacactatag 20
<210> 6
   <211> 20
   <212> DNA
   <213> Artificial Sequence
<220>
   <223> Synthetic Primer
<400> 6
   taatacgact cactataggg 20

## Description

### Cross-Reference to Related Applications

This application claims priority to United States provisional patent application number 60/896,509 filed March 23, 2007.

### Field of the Invention

The methods disclosed relate to improved processes for DNA amplification by multiply primed rolling circle amplification so as to provide higher purity products.

### Background of the Invention

Several useful methods have been developed that permit amplification of nucleic acids. Most were designed around the amplification of selected DNA targets and/or probes, including the polymerase chain reaction (PCR), ligase chain reaction (LCR), self-sustained sequence replication (3SR), nucleic acid sequence based amplification (NASBA), strand displacement amplification (SDA), and amplification with Qβ replicase (Birkenmeyer and Mushahwar, J. Virological Methods, 35:117-126 (1991)); (Landegren, Trends Genetics, 9:199-202 (1993)). In addition, several methods have been employed to amplify circular DNA molecules such as plasmids or DNA from bacteriophage such as M13. One application has been propagation of these molecules in suitable host bacteria such as strains of *E. coli,* followed by isolation of the DNA by well-established protocols (Sambrook, J., Fritsch, E. F., and Maniatis, T. Molecular Cloning, A Laboratory Manual, 1989, Cold Spring Harbor Laboratory Press, Cold Spring Harbor, N.Y.). PCR has also been a frequently used method to amplify defined sequences in DNA targets such as plasmids and DNA from bacteriophage such as M13. Some of these methods suffer from being laborious, expensive, time-consuming, inefficient, and lacking in sensitivity. They may also require specific knowledge about the sequences to be amplified.

As an improvement on these methods, linear rolling circle amplification (LRCA) uses a primer annealed to a circular target DNA molecule and DNA polymerase is added. The amplification target circle (ATC) forms a template on which new DNA is made, thereby extending the primer sequence as a continuous sequence of repeated sequences complementary to the circle but generating only about several thousand copies per hour. An improvement on LRCA is the use of exponential RCA (ERCA), with additional primers that anneal to the replicated complementary sequences to provide new centers of amplification, thereby providing exponential kinetics and increased amplification. Exponential rolling circle amplification (ERCA) employs a cascade of strand displacement reactions, also referred to as HRCA (Lizardi, P. M. et al. Nature Genetics, 19, 225-231 (1998)).

In US 6,323,009, a means of amplifying target DNA molecules is introduced. This method is of value because such amplified DNA is frequently used in subsequent methods including DNA sequencing, cloning, mapping, genotyping, generation of probes for hybridization experiments, and diagnostic identification.

The methods of the US 6,323,009 patent (referred to herein as Multiply Primed Amplification--MPA) improve the sensitivity of linear rolling circle amplification by using multiple primers for the amplification of individual target circles. The MPA method has the advantage of generating multiple tandem-sequence DNA (TS-DNA) copies from each circular target DNA molecule. In addition, MPA has the advantages that in some cases the sequence of the circular target DNA molecule may be unknown while the circular target DNA molecule may be single-stranded (ssDNA) or double-stranded (dsDNA or duplex DNA). Another advantage of the MPA method is that the amplification of single-stranded or double-stranded circular target DNA molecules may be carried out isothermally and/or at ambient temperatures. Other advantages include being highly useful in new applications of rolling circle amplification, low cost, sensitivity to low concentration of target circle, flexibility, especially in the use of detection reagents, and low risk of contamination.

The MPA method can improve on the yield of amplified product DNA by using multiple primers that are resistant to degradation by exonuclease activity that may be present in the reaction. This has the advantage of permitting the primers to persist in reactions that contain an exonuclease activity and that may be carried out for long incubation periods. The persistence of primers allows new priming events to occur for the entire incubation time of the reaction, which is one of the hallmarks of ERCA and has the advantage of increasing the yield of amplified DNA.

The MPA method allows for the first time *"in vitro* cloning", i.e. without the need for cloning into an organism, of known or unknown target DNAs enclosed in circles. A padlock probe may be used to copy the target sequence into a circle by the gap fill-in method (Lizardi, P. M. et al. Nature Genetics, 19,225-231 (1998)). Alternatively, target sequences can be copied or inserted into circular ssDNA or dsDNA by many other commonly used methods. The MPA amplification overcomes the need to generate amplified yields of the DNA by cloning in organisms such as bacterial host cells.

The MPA method is an improvement over LRCA in allowing increased rate of synthesis and yield. This results from the multiple primer sites for DNA polymerase extension. Random primer MPA also has the benefit of generating double stranded products. This is because the linear ssDNA products generated by copying of the circular template will themselves be converted to duplex form by random priming of DNA synthesis. Double stranded DNA product is advantageous in allowing for DNA sequencing of either strand and for restriction endonuclease digestion and other methods used in cloning, labeling, and detection.

It is also expected that strand-displacement DNA synthesis may occur during the MPA method resulting in an exponential amplification. This is an improvement over conventional ERCA, also termed HRCA (Lizardi et al. (1998)) in allowing for the ability to exponentially amplify very large linear or circular DNA targets. The amplification of large circular DNA, including bacterial artificial chromosomes (BACs), has been reduced to practice using the MPA method.

Methods have published for whole genome amplification using degenerate primers (Cheung, V. G. and Nelson, S. F. Proc. Natl. Acad. Sci. USA, 93, 14676-14679 (1996)) and random primers (Zhang, L. et al., Proc. Natl. Acad. Sci. USA, 89, 5847-5851 (1992)) where a subset of a complex mixture of targets such as genomic DNA is amplified. Reduction of complexity is an objective of these methods. A further advantage of the MPA method is that it amplifies DNA target molecules without the need for "subsetting", or reducing the complexity of the DNA target.

The MPA method rapidly amplifies every sequence within the sample of DNA used with it, the double-stranded product has all the same sequences as the original sample. Except for the fact that it contains tandemly-repeated copies of the DNA with numerous initiation (priming) sites, the physical properties of the product DNA are much like those of the starting template.

It has been found that when a mixture of long, linear and circular DNAs are provided for MPA, sequences within both forms are amplified. However, the majority of cloned DNAs grown in bacterial and other hosts are circular such as the DNA found in plasmids, bacterial artificial chromosomes (BACs), fosmids, cosmids, certain bacteriophage clones such as M13 clones and others. It is common to seek to isolate the clone sequences separate from those of the host cells for further analysis. It should be noted that host cell chromosomes are much larger than BAC clones and are nearly always isolated as broken linear fragments of the circular chromosome.

Accordingly, there is a need for amplification methods that lack the limitations of PCR and which favor amplification of circular forms of DNA over long, linear forms. These concerns are addressed in greater detail below.

### Nucleases

Nucleic acids such as DNA or RNA consist of chains of 2 or more nucleotides held together by phosphodiester linkages. They can be either single chains (single stranded) or they may have two complementary strands not covalently attached to each other that pair in opposite orientations (double stranded). Nucleotides are held together in a nucleic acid chain by phosphodiester linkages between the 3' hydroxyl of one nucleoside and the 5' hydroxyl of its neighbor. An end of a nucleic acid chain can be distinguished as a 3' end or a 5' end depending on whether it has a 3' or 5' hydroxyl that is not joined to another nucleotide. These hydroxyl groups are often found phosphorylated. For the most part, naturally occurring nucleic acids have one 5' end and one 3' end.

Nucleases are enzymes that catalyze the hydrolysis of phosphodiesters that are part of the backbone of nucleic acids, particularly RNA and DNA. Like their nucleic acid substrates, they are found in nature with a variety of specificities and activities. Thus some nucleases may hydrolyze RNA much faster than DNA or hydrolyze single stranded DNA much faster than double stranded DNA, Other enzymes may work almost equally well on both single- and double-stranded DNA. Generally, the nucleases are named according to the nucleic acid substrate hydrolyzed fastest for that particular enzyme.

### Endonucleases

Endonucleases are enzymes that catalyse the hydrolysis of the phosphodiester backbone of nucleic acids from internal sites of the polymer chain. They do not require a free end of the polymer as substrate.

### Exonucleases

Exonucleases are enzymes that catalyse the hydrolysis of the phosphodiester backbone of nucleic acids from the end of the polymer. Many exonucleases hydrolyze nucleotides either from only 3' or from only 5' ends. Involved in recombination, repair, replication, and the editing and processing of DNA and RNA, this class of enzymes encompasses a large number of different specificities and functions (Linn and Roberts, 1982, Linn et al., 1993).

### Single Stranded Exonucleases

Single-stranded (ss) exonucleases hydrolyze nucleic acids that consist of a single nucleic acid polymer chain.

### Double Stranded Exonucleases

Double-stranded (ds) exonucleases hydrolyze nucleic acids that consist of two based-paired nucleic acid polymer strands.

### Table of Exonucleases

Examples of ss Exonucleases include, but are not limited to those listed in Table I.

### E. coli Exo I

E. coli Exonuclease I (Exo I) is a single strand exonuclease that hydrolyses DNA in a 3' to 5' direction from a free 3' hydroxyl end. This enzyme is highly processive, making it suitable for hydrolyzing either short or long stretches of ssDNA.

### E coli. Exo III

E.coli Exonuclase III (Exo III) is a non-processive 3'-5' double strand exonuclease.

### Polymerase 3-5 exo

Many DNA polymerases, for example bacteriophage Phi 29 DNA polymerase, have an intrinsic 3'-5' exonuclease activity that acts on the 3' end of double stranded DNA. This activity provides the "proofreading" ability of many DNA polymerases, removing mistakenly incorporated nucleotides and thereby increasing fidelity. It can also be employed to degrade DNA in a 3'-5' direction on a single strand of DNA that is either single- or double-stranded.

A mixture of exonuclease I and exonuclease III has been used in the preparation of plasmid DNA from E.coli (Hyman, BioTechniques 13 550 (1992)).

WO02/38755A relates to novel circular extra-chromosomal DNA elements. It provides a method for purifying such DNA elements from animal tissue, which may include a step of treating the isolated DNA element with lambda exonuclease to remove residual linear DNA. The isolated circular DNA may be PCR amplified.

Gaubatz J W et al: "Purification of eukaryotic extrachromosomal circular DNAs using exonuclease III", 19900101. Vol. 184, no 2, January 1990, pages 305-310, XP001024672, relates to a method for purifying circular extra-chromosomal DNA elements from animal tissue, which includes a step of treating the isolated DNA element with exonuclease III to remove linear DNA. The isolated circular DNA are analyzed using restriction and other DNA modifying enzymes.

Dean F B et al: "Rapid amplification of plasmid and phage DNA using Phi29 NDA polymerase and multiply-primed rolling circle amplification" Genome Research, Cold Spring Harbor Laboratory Press, Woodbury, NY, US, vol 11, no 6,1 June 2001, pages 1095-1099, XP00222317 ISSN:1088-9051, relates to amplification of plasmid and phage DNA, directly from single colonies or plaques, using phi29 DNA polymerase directed multiple-primed rolling circle amplification.

Nelson J R et al: "Templiphi, Phi29 DNA polymerase based rolling circle amplification of templates for DNA sequencing", Biotechniques, Informa Life Sciences Publishing, Westborough, MA, US, 1 June 2002, pages S44-S47, XP001204520 ISSN: 0736-6205, relates to the generation of templates for DNA sequencing, using circular DNA as starting material. Plasmid DNA is amplified directly from E coli colony or liquid culture, using TempliPhi, phi29 based isothermal amplification kit.

Hyman E D: "Preparation of plasmid DNA by sequential enzymatic digestion", Biotechniques, vol 13, no 4,1992, pages 550, 552-554, XP8165156, ISSN: 0736-6205, relates to the preparation of plasmid DNA from E coli by a multi-step purification process. In order to remove chromosomal DNA, exonucleases are added as one of the steps, to hydrolyze the linear DNA.

### Summary of the Invention

A new method of amplification is disclosed that can be used for circular DNA can provide for complete or near-complete elimination of DNA sequences originally present in linear form, and which is carried out isothermally. This is achieved by treatment of the template mixture with two or more exonucleases prior to amplification. The resulting amplified product may be used for sequencing or restriction endonuclease digestion and fingerprinting or for other downstream analysis purposes.

The DNA sequences to be amplified should be in a circular form. It is preferably in double stranded form although single stranded circular DNA can be used. The circular DNA can be mitochondrial DNA, plasmid, fosmid, bacteriophage, viral or bacterial artificial chromosome. The circular DNA may have been modified by the insertion of a DNA sequence. The inserted DNA sequence may have a known sequence or may have an unknown sequence. The circular DNA may also be formed by ligation of linear DNA. It may be necessary in this case to dilute the resulting circular DNA to a level where on average only one circular DNA molecule is initially present in the subsequent amplification reaction. The circular DNA to be amplified may also be made by using a ligase to close any nicks present in the circular DNA. It is preferred that the method of amplification is Rolling Circle Amplification (RCA).

The methods disclosed describe a process for the enhanced amplification of DNA targets using either specific or random primers. In one aspect multiple primers are used (specific or random, exonuclease-sensitive or exonuclease-resistant) annealed to the target DNA molecules to increase the yield of amplified product from RCA. Multiple primers anneal to multiple locations on the target DNA and extension by polymerase is initiated from each location. In this way multiple extensions are achieved simultaneously from the target DNA. The extension process can be carried out in the presence of one or more nucleotide analogs, optionally in the presence of all four normal nucleotides. The nucleotide analogs confer unusual properties to the product DNA without changing its sequence content.

The use of multiple primers is achieved in several different ways. It is achieved by using two or more specific primers that anneal to different known sequences on the target DNA, or by having one given primer anneal to a sequence repeated at two or more separate locations on the target DNA, or by using random or degenerate sequence primers, which can anneal to many locations on the target DNA.

In some methods, the primers for MPA contain nucleotides, including modified nucleotides, which may serve to make the primers resistant to nuclease enzyme degradation. Enzyme degradation may be caused by a specific exonuclease such as the 3'-5' exonuclease activity associated with DNA polymerase or by a contaminating exonuclease.

The various cloning vectors which create circular forms of a DNA clone within host cells often differ in the number of copies found within each cell. While some advantageous plasmid vectors may replicate to the point where they create hundreds or even thousands of copies per cell, they are often limited to carrying only a limited size of DNA up to perhaps only 5 Kb. Other vectors such as cosmid vectors, readily carry about 40-50 Kb of sequence, but have limited numbers of copies in each cell. The vectors that carry the longest sequences such as BAC vectors, limit themselves to a single copy per cell, making the sequences desired for analysis a much smaller fraction of the total DNA derived from both the BAC and the host chromosome. Amplification by ordinary MPA does little to improve this ratio since it amplifies all sequences whether present in linear or circular form to similar extents. The methods described introduce a treatment with exonuclease to enrich circular sequences presented to the MPA process, greatly improving results obtained downstream. This nuclease treatment can be readily carried out in the same container as the amplification since the nuclease or nucleases used can be inactivated by heat treatment.

### Brief Description of the Drawings

FIG. 1 is a schematic representation of the method applied to amplification of circular DNA from a cell. The host cell DNA is large compared with the circular DNA. The host cell DNA may be capped with special telomeric structures.
FIG. 2 is a representation of the method using linear DNA and amplification of specific sequences in the linear DNA.
FIG. 3 is a representation of the method using circular DNA which may contain nicks.
FIG. 4 is a representation of the method using in vitro cloned DNA.

### Detailed Description of the Invention

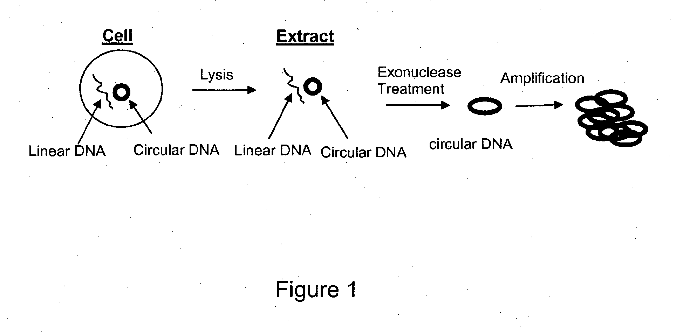

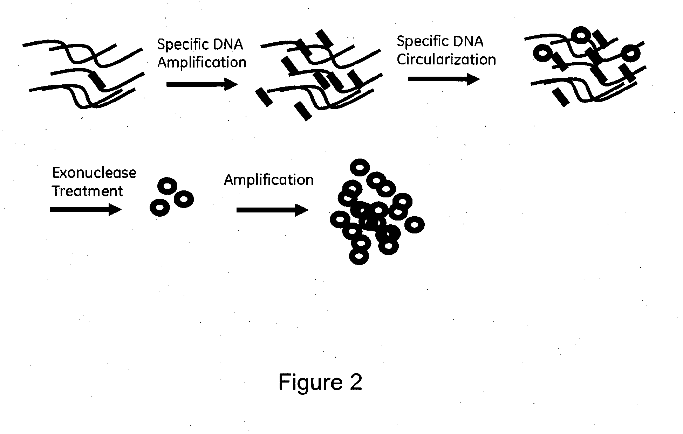

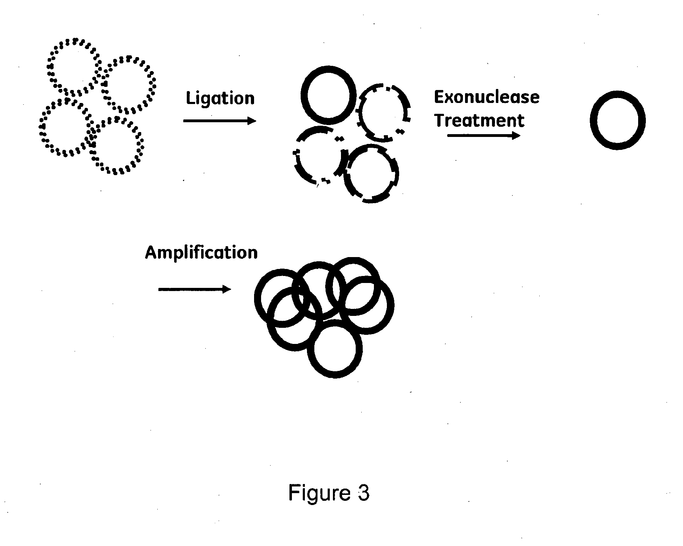

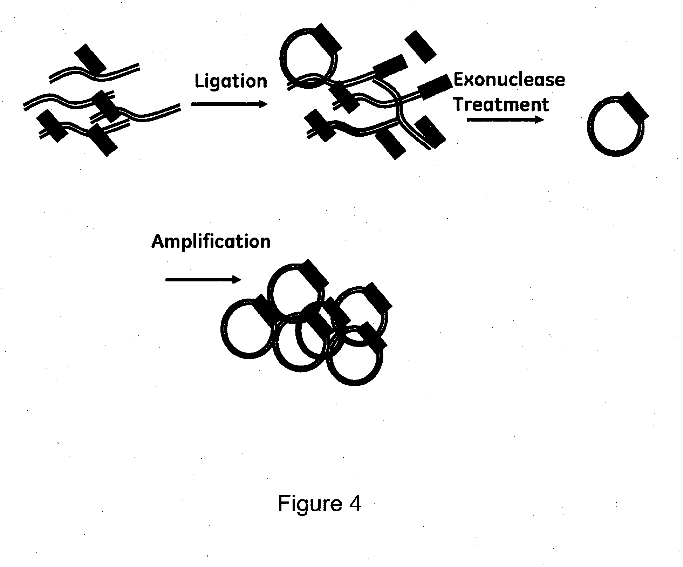

In FIG. 1 - 4 the final amplification product consists of linear tandem-repeat copies of the original circle sequence and some identical circular copies of the original circle sequence.

The methods disclosed relate to analysis of DNA and in particular to analyses that depend on the sequence of DNA, often used for determining genotype as well as original sequence information. It also pertains to amplification of DNA sequences. Amplification means synthesis of new strands of DNA which have complimentary sequence to the original, preserving the original sequence information. While some amplification methods such as polymerase chain reaction (PCR) are highly specific and yield amplified products of defined length, others are general, amplifying all the DNA sequences present in a sample yielding products that vary in length yet still contain the original sequence information. An example of this latter kind of amplification is MPA as described in US 6,323,009.

The methods disclosed also relate to DNA sequencing. One method of DNA sequencing is the Sanger or dideoxy method which is defined as a method for determining the nucleotide base sequence of a DNA molecule comprising the steps of incubating the nucleic acid molecule with an oligonucleotide primer, a plurality of deoxynucleoside triphosphates, at least one chain terminating agent, and a DNA polymerase under conditions in which the primer is extended until the chain terminating agent is incorporated. The products are separated according to size, detected and whereby at least a part of the nucleotide base sequence of the original DNA molecule can be determined (see, for example US 5,639,608).

A more advantageous sequencing method is cycle sequencing with dideoxynucleotide terminators. Cycle sequencing involves multiple rounds of DNA synthesis carried out from the same template using an oligonucleotides primer. The newly synthesized strand is removed from the template strand after each synthesis cycle by heat denaturation; this amplifies the number of strands produced in the sequencing process and allows much smaller amounts of DNA template to be sequenced (US 5,614,365). A particularly useful way of performing cycle sequencing is with thermally stable DNA polymerase and fluorescent-labeled dideoxynucleotide terminators (for example US 5,366,860). This, most popular method of sequencing typically makes use of dITP to eliminate electrophoresis artifacts, and four distinct fluorescent labels for the four nucleotide bases.

The polymerase chain reaction (PCR) is defined as a process for amplifying at least one specific nucleic acid sequence contained in a nucleic acid or a mixture of nucleic acids wherein each nucleic acid consists of two separate complementary strands. First, the strands are combined with two oligonucleotide primers, for the specific sequence being amplified, under conditions such that the extension product synthesized from one primer, when it is separated from its complement, can serve as a template for synthesis of the extension product of the other primer. The primers are extended using DNA polymerase then the extension products denatured by heating from the templates on which they were synthesized to produce single-stranded molecules. Upon cooling to an annealing temperature, the single-stranded molecules generated anneal with the primers and are again extended by DNA polymerase. The process is repeated one or more times resulting in exponential amplification of the sequences "between" the priming sites US 4,683,202.

Single Strand Confirmation Polymorphism (SSCP) is a process that can be used for the detection of polymorphisms (Orita et al, PNAS 86(8) April 1989 2766-70; Less-et al. Mol Eco12(2) p. 119-29 April 1993). Essentially, labeled, denatured fragments of DNA are applied to a non-denaturing electrophoresis gel. If polymorphisms (sequence variants) exist in the fragment, more than one band may be observed on the gel because the conformation of the single-stranded fragments differ with different sequences.

Hybridization is a technique of using the natural tendency for nucleic acids to bind specifically to other nucleic acid strands with complimentary sequence. Virtually all molecular biology experiments feature hybridization, for example the sequencing primer hybridizes with the sequencing template. Similarly the PCR primers hybridize with the desired template strands. More general hybridization experiments may involve hybridization of an immobilized nucleic acid with a soluble, labeled or tagged nucleic acid in techniques variously called "Southern" hybridizations (Southern, E., J Mol Biol. 1975 98(3):503-17), "Northern" hybridizations (Alwine, et al., Proc Natl Acad Sci USA. 1977; 74(12):5350-4) and more recent microarray hybridizations (see for example WO 92/10588).The methods described relates to the use of multiple primers in nucleic acid sequence amplification as a means of greatly amplifying DNA synthesis and providing greatly increased amounts of DNA for detection of specific nucleic acid sequences contained in, for example, a target DNA. While previous methods have often employed targets of substantial complexity, the methods disclosed herein utilize relatively simple targets, such as simple plasmid, cosmid and bacterial artificial chromosome (BAC) targets.

In some methods a premix can be used, such as in the form of a kit, comprising a polymerase, even including more than one polymerase, one or more exonucleaseses, nuclease-protected oligonucleotide primers, such as random-sequence hexamers, the required nucleoside triphosphates, an appropriate buffer, optionally a pyrophosphatase, and other potentially desirable components, either with each such component in a separate vial or mixed together in different combinations so as to form a total of one, two, three, or more separate vials and, for example, a blank or buffer vial for suspending an intended target nucleic acid for use in the amplification process. One kit for amplifying DNA sequences comprises nuclease-resistant random primers, a DNA polymerase and the four deoxyribonucleoside triphosphates (dNTPs). The DNA polymerase can have 3'-5' exonuclease activity. The DNA polymerase can be Φ29 DNA polymerase. In some methods, at least one of the normal dNTPs is replaced, in whole or in part, by an analog whose presence in the product DNA confers some advantageous property to said product DNA or to subsequent processes such as sequence-dependent analyses. In a specific application, there is provided a process whereby a sample of nucleic acid, such as a DNA, is suspended in a buffer, such as TE buffer, and then treated with one or more exonucleases and then optionally heated, cooled, and then contacted with the remaining components recited above, either sequentially or by adding such components as the aforementioned premix with the conditions of temperature, pH and the like subsequently adjusted, for example by maintaining such combination at 10°C.

In addition, the conditions used in carrying out the processes disclosed may vary during any given application. Thus, by way of non-limiting example, the primers and target DNA may be added under conditions that promote hybridization and the DNA polymerase and nucleoside triphosphates added under different conditions that promote amplification without causing denaturation of the primer-target complexes that act as substrates for the polymerase or polymerases.

In some of the methods disclosed the target DNA binds to, or hybridizes to, at least 3, 4, 5, even 10, or more primer oligonucleotides, each said primer producing, under appropriate conditions, a separate tandem-repeat sequence DNA molecule. Because the sequences of the tandem sequence DNAs (TS-DNAs) are complementary to the sequences of the target DNA, which act as template, the TS-DNA products will all have the same sequence as the target DNA, regardless of the sequence of the primers and the nucleotide content of the TS-DNA product will be determined by the mixture of nucleotides or nucleotide analogs used for the amplification subject to the selective power of the DNA polymerase or polymerases used in the amplification process.

The oligonucleotide primers useful in the processes of amplification can be of any desired length. For example, such primers may be of a length of from at least 2 to about 30 to 50 nucleotides long, preferably about 2 to about 35 nucleotides in length, most preferably about 5 to about 10 nucleotides in length, with hexamers and octamers being specifically preferred embodiments. Such multiple primers as are used herein may equally be specific only, or random-sequence only, or a mixture of both, with random primers being especially useful and convenient to form and use.

Amplification target DNA useful in the processes disclosed are DNA or RNA molecules, either single or double stranded, including DNA-RNA hybrid molecules generally containing between 40 to 500,000 nucleotides. It can be preferable that the DNA is the range of 200 to 200,000 nucleotides. However, it is expected that there will be no upper limit to the size of the target, particularly when using short, random-sequence primers. Where the target is a duplex, such numbers are intended to refer to base pairs rather than individual nucleotide residues. The target templates useful in the processes disclosed herein may have functionally different portions, or segments, making them particularly useful for different purposes. At least two such portions will be complementary to one or more oligonucleotide primers and, when present, are referred to as a primer complementary portions or sites. Amplification targets useful in the methods disclosed include, for example, those derived directly from such sources as a bacterial colony, a bacteriophage, a virus plaque, a yeast colony, a baculovirus plaque, as well as native or transiently transfected eukaryotic cells. Such sources may or may not be lysed prior to obtaining the targets. Where such sources have been lysed, such lysis is commonly achieved by a number of means, including where the lysing agent is heat, an enzyme, the latter including, but not limited to, enzymes such as lysozyme, helicase, glucylase, and zymolyase, or such lysing agent may be an organic solvent or a solution of high pH.

In MPA, amplification occurs with each primer, thereby forming a concatemer of tandem repeats (i.e., a TS-DNA) of segments complementary to the primary ATC (or ATC) being replicated by each primer. Thus, where random primers are used, many such TS-DNAs are formed, one from each primer, to provide greatly increased amplification of the corresponding sequence since the nucleotide sequence, or structure, of the product depends only on the sequence of the template and not on the sequences of the oligonucleotide primers, whether the latter are random or specific or a mixture of both.

The amplification method disclosed herein is distinct from published modified PCR methods (see for example Cheung, V. G. and Nelson, S. F. Proc. Natl. Acad. Sci. USA, 93, 14676-14679 (1996) and Zhang, L. et al., Proc. Natl. Acad. Sci. USA, 89, 5847-5851 (1992)) by facilitating use of random or multiple primers in an amplification of linear DNA target with a DNA polymerase, such as Φ29 DNA polymerase as a preferred enzyme for this reaction, along with exonuclease-resistant primers (as described below). Therefore, the present disclosure includes a method for the amplification of linear DNA targets, including high molecular weight DNAs, as well as genomic and cDNAs, that takes advantage of the characteristics of Φ29 DNA polymerase and the exonuclease-resistant primers that are compatible with the 3'-5' exonuclease activity associated with Φ29 DNA polymerase and wherein said linear DNA target may be used instead of or in addition to circular DNA.

Where duplex circles are employed, amplification will commonly occur from both strands as templates. Simultaneous amplification of both circles may or may not be desirable. In cases where the duplex circles are to be further employed in reactions designed to sequence the DNA of said circles, amplification of both strands is a desirable feature and so the duplex circles can be directly employed without further processing (except for formation of a nick if needed).

In some circumstances it may be desirable to quantitatively determine the extent of amplification occurring. In such instances, the amplification step of the present methods work well with any number of standard detection schemes, such as where special deoxynucleoside triphosphates (dNTPs) are utilized that make it easier to do quantitative measurements. The most common example is where such nucleotide substrates are radiolabeled or have attached thereto some other type of label, such as a fluorescent label or the like. These are typically used in trace amounts so as to minimally disturb the composition of the product DNA. Again, the methods that can be employed in such circumstances are many and the techniques involved are standard and well known to those skilled in the art. Thus, such detection labels include any molecule that can be associated with amplified nucleic acid, directly or indirectly, and which results in a measurable, detectable signal, either directly or indirectly. Many such labels for incorporation into nucleic acids or coupling to nucleic acid probes are known to those of skill in the art. General examples include radioactive isotopes, fluorescent molecules, phosphorescent molecules, enzymes, antibodies, and ligands.

Examples of suitable fluorescent labels include Cy Dyes such as Cy2, Cy3, Cy3.5, Cy5, and Cy5.5, available from GE Healthcare (US 5,268,486). Further examples of suitable fluorescent labels include fluorescein, 5,6-carboxymethyl fluorescein, Texas red, nitrobenz-2-oxa-1,3-diazol-4-yl (NBD), coumarin, dansyl chloride, and rhodamine. Preferred fluorescent labels are fluorescein (5-carboxyfluorescein-N-hydroxysuccinimide ester) and rhodamine (5,6-tetramethyl rhodamine). These can be obtained from a variety of commercial sources, including Invitrogen and Research Organics, Cleveland, Ohio.

Labeled nucleotides are a preferred form of detection label since they can be directly incorporated into the products of amplification during synthesis. Examples of detection labels that can be incorporated into amplified DNA include nucleotide analogs and nucleotides modified with biotin (Langer et al., Proc. Natl. Acad. Sci. USA, 78:6633 (1981)) or with suitable haptens such as digoxygenin (Kerkhof, Anal. Biochem., 205:359-364 (1992)). Suitable fluorescence-labeled nucleotides are Fluorescein-isothiocyanate-dUTP, Cyanine-3-dUTP and Cyanine-5-dUTP (Yu et al., Nucleic Acids Res., 22:3226-3232 (1994)). A preferred nucleotide analog detection label for DNA is BrdUrd (BUDR triphosphate, Sigma), and a preferred nucleotide analog detection label is Biotin-16-uridine-5'-triphosphate (Biotin-16-dUTP, Boehringher Mannheim). Radiolabels are especially useful for the amplification methods disclosed herein.

The methods disclosed provide high amplification rates due to multiple priming events being induced on molecules that are targets for amplification. Thus, the rate and extent of amplification is not limited to that accomplished by a single DNA polymerase copying the DNA circle. Instead, multiple DNA polymerases are induced to copy each template circle simultaneously, each one initiating from one of the primers.

Exonuclease-resistant primers useful in the methods disclosed herein may include modified nucleotides to make them resistant to exonuclease digestion. For example, a primer may possess one, two, three or four phosphorothioate linkages between nucleotides at the 3' end of the primer.

The amplification step can include a process wherein the primers contain at least one nucleotide that makes the primer resistant to degradation, commonly by an enzyme, especially by an exonuclease and most especially by 3'-5'-exonuclease activity. In such an embodiment, at least one nucleotide may be a phosphorothioate nucleotide or some modified nucleotide. Such nucleotide is commonly a 3'-terminal nucleotide but the processes described here also relate to methods where such a nucleotide is located at other than the 3'-terminal position and wherein the 3'-terminal nucleotide of said primer can be removed by 3'-5'-exonuclease activity. The dNTPs used in the amplification may also be nuclease resistant.

Attachment of target templates or oligonucleotide primers to solid supports may be advantageous and can be achieved through means of some molecular species, such as some type of polymer, biological or otherwise, that serves to attach said primer or target template to a solid support. Such solid-state substrates useful in the methods described can include any solid material to which oligonucleotides can be coupled. This includes materials such as acrylamide, cellulose, nitrocellulose, glass, polystyrene, polyethylene vinyl acetate, polypropylene, polymethacrylate, polyethylene, polyethylene oxide, glass, polysilicates, polycarbonates, teflon, fluorocarbons, nylon, silicon rubber, polyanhydrides, polyglycolic acid, polylactic acid, polyorthoesters, polypropylfumerate, collagen, glycosaminoglycans, and polyamino acids. Solid-state substrates can have any useful form including thin films or membranes, beads, bottles, dishes, fibers, woven fibers, shaped polymers, particles and microparticles. A preferred form for a solid-state substrate is a glass slide or a microtiter dish (for example, the standard 96-well dish). Preferred embodiments utilize glass or plastic as the support. For additional arrangements, see those described in US 5,854,033.

Methods for immobilization of oligonucleotides to solid-state substrates are well established. Oligonucleotides, including address probes and detection probes, can be coupled to substrates using established coupling methods. For example, suitable attachment methods are described by Pease et al., Proc. Natl. Acad. Sci. USA 91(11):5022-5026 (1994). A preferred method of attaching oligonucleotides to solid-state substrates is described by Guo et al., Nucleic Acids Res. 22:5456-5465 (1994).

Oligonucleotide primers can be synthesized using established oligonucleotide synthesis methods. Methods of synthesizing oligonucleotides are well known in the art. Such methods can range from standard enzymatic digestion followed by nucleotide fragment isolation (see for example, Sambrook, et al., Molecular Cloning: A Laboratory Manual, Second Edition, Cold Spring Harbor, N.Y., (1989), Wu et al., Methods in Gene Biotechnology (CRC Press, New York, N.Y., 1997), and Recombinant Gene Expression Protocols, in Methods in Molecular Biology, Vol. 62, (Tuan, ed., Humana Press, Totowa, N.J., 1997)) to purely synthetic methods, for example, by the cyanoethyl phosphoramidite method using a Milligen or Beckman System 1 Plus DNA synthesizer (for example, Model 8700 automated synthesizer of Milligen-Biosearch, Burlington, Mass. or ABI Model 380B). Synthetic methods useful for making oligonucleotides are also described by Ikuta et al., Ann. Rev. Biochem. 53:323-356 (1984), (phosphotriester and phosphite-triester methods), and Narang et al., Methods in Enzymology, 65:610-620 (1980), (phosphotriester method). Protein nucleic acid molecules can be made using known methods such as those described by Nielsen et al., Bioconjugate. Chem. 5:3-7 (1994).

Methods for the synthesis of primers containing exonuclease-resistant phosphorothioate diesters by chemical sulfurization are well-established. The solid phase synthesis of random primers employs one or several specifically placed internucleotide phosphorothioate diesters at the 3'-end. Phosphorothioate triesters can be introduced by oxidizing the intermediate phosphite triester obtained during phosphoramidite chemistry with 3H-1, 2-benzodithiol-3-one 1,1 dioxide.sup.1,2 or Beaucage reagent to generate pentavalent phosphorous in which the phosphorothioate triester exists as a thione. The thione formed in this manner is stable to the subsequent oxidation steps necessary to generate internucleotidic phosphodiesters. (Iyer, R. P., Egan, W., Regan, J. B., and Beaucage, S. L. J. Am. Chem. Soc., 112: 1253 (1990) and Iyer, R. P., Philips, L. R., Egan, W., Regan, J. B., and Beaucage, S. L. J. Org. Chem., 55: 4693 (1990)).

Many of the oligonucleotides described herein are designed to be complementary to certain portions of other oligonucleotides or nucleic acids such that hybrids can be formed between them. The stability of these hybrids can be calculated using known methods such as those described in Lesnick and Freier, Biochemistry 34:10807-10815 (1995), McGraw et al., Biotechniques 8:674-678 (1990), and Rychlik et al., Nucleic Acids Res. 18:6409-6412 (1990).

DNA polymerases useful in the isothermal amplification step are referred to herein as amplification DNA polymerases. For amplification, it is preferred that a DNA polymerase be capable of displacing the strand complementary to the template strand, termed strand displacement, and lack a 5' to 3' exonuclease activity. Strand displacement is necessary to result in synthesis of multiple tandem copies of the target template. A 5' to 3' exonuclease activity, if present, might result in the destruction of the synthesized strand. It is also preferred that DNA polymerases for use in the disclosed method are highly processive. The suitability of a DNA polymerase for use in the disclosed method can be readily determined by assessing its ability to carry out amplification. Preferred amplification DNA polymerases, all of which have 3', 5'-exonuclease activity, are bacteriophage. Φ29 DNA polymerase (US 5,198,543 and US 5,001,050 to Blanco et al.), phage M2 DNA polymerase (Matsumoto et al., Gene 84:247 (1989)), phage PRD1 DNA polymerase (Jung et al., Proc. Natl. Aced. Sci. USA 84:8287 (1987), and Zhu and Ito, Biochim. Biophys. Acta. 1219:267-276 (1994)), VENT.TM. DNA polymerase (Kong et al., J. Biol. Chem. 268:1965-1975 (1993)), Klenow fragment of DNA polymerase I (Jacobsen et al., Eur. J. Biochem. 45:623-627 (1974)), T5 DNA polymerase (Chatterjee et al., Gene 97:13-19 (1991)), and T4 DNA polymerase holoenzyme (Kaboord and Benkovic, Curr. Biol. 5:149-157 (1995)). Φ29 DNA polymerase is most preferred. Equally preferred polymerases include native T7 DNA polymerase, Bacillus stearothermophilus (Bst) DNA polymerase, Thermoanaerobacter thermohydrosulfuricus (Tts) DNA polymerase (US 5,744,312), and the DNA polymerases of Thermus aquaticus, Thermus flavus or Thermus thermophilus. Equally preferred are the Φ29-type DNA polymerases, which are chosen from the DNA polymerases of phages: Φ29, Cp-1, PRD1, Φ15, Φ21, PZE, PZA, Nf, M2Y, B103, SF5, GA-1, Cp-5, Cp-7, PR4, PR5, PR722, and L17. In a specific embodiment, the DNA polymerase is bacteriophage. Φ29 DNA polymerase wherein the multiple primers are resistant to exonuclease activity and the target DNA is linear DNA, especially high molecular weight and/or complex linear DNA, genomic DNA, cDNA.

Strand displacement during amplification, especially where duplex target templates are utilized as templates, can be facilitated through the use of a strand displacement factor, such as a helicase. In general, any DNA polymerase that can perform amplification in the presence of a strand displacement factor is suitable for use in the processes described herein, even if the DNA polymerase does not perform amplification in the absence of such a factor. Strand displacement factors useful in amplification include BMRF1 polymerase accessory subunit (Tsurumi et al., J. Virology 67(12):7648-7653 (1993)), adenovirus DNA-binding protein (Zijderveld and van der Vliet, J. Virology 68(2):1158-1164 (1994)), herpes simplex viral protein ICP8 (Boehmer and Lehman, J. Virology 67(2):711-715 (1993); Skaliter and Lehman, Proc. Natl, Acad. Sci. USA 91(22):10665-10669 (1994)), single-stranded DNA binding proteins (SSB; Rigler and Romano, J. Biol. Chem. 270:8910-8919 (1995)), and calf thymus helicase (Siegel et al., J. Biol Chem. 267:13629-13635 (1992)).

The ability of a polymerase to carry out amplification can be determined by testing the polymerase in a rolling circle replication assay such as those described in Fire and Xu, Proc. Natl. Acad. Sci. USA 92:4641-4645 (1995) and in Lizardi (US 5,854,033, e.g., Example 1 therein).

The target DNA may be, for example, a single stranded bacteriophage DNA or double stranded DNA plasmid or other vector, which is amplified for the purpose of DNA sequencing, cloning or mapping, and/or detection. The examples below provide specific protocols but conditions can vary depending on the identity of the DNA to be amplified and analyzed or sequenced.

In US 6,323,009, a means of amplifying target DNA molecules is described. Some embodiments of this method feature the use of random-sequence hexamer primers added in great excess to target DNA, Φ29 DNA polymerase and the four normal dNTPs (dATP, dCTP, dGTP and dTTP) to produce multiple copies of all the sequences present in the original target sample. One way of checking that the product is similar to the starting target is to measure the Tm of both the product and the starting target template. Another is to use restriction endonucleases to digest the product DNA and the original target DNA and compare the sizes of the digestion products by gel electrophoresis. Similarly, sequence analysis can be performed on both the target and product DNAs.

In cases where such comparisons have been made, the Tm, restriction digest and sequence information clearly indicated that the product DNA is the same as the starting target DNA in the parameters that can usually be measured by these methods. Thus, while the overall molecular size of the product DNA may apparently be much larger than the starting target DNA, its restriction digestion pattern, melting temperature and sequence are the same.

While it has been found that DNA sequencing of certain types of templates is improved by the methods described, this is just one example of an analysis method that relies on the hybridization of nucleic acid strands for its functionality. During the sequencing process, the primer must hybridize with its template, and the newly-synthesized strand must remain hybridized with its template strand in order to give a useful result. Many other methods of analysis rely on hybridization steps. These include hybridizations performed on solid surfaces such as Southern- and Northern-hybridizations, hybridizations on arrays and micro-arrays. They also include amplification by polymerase chain reaction (PCR) which itself can be used for genotyping and other analyses. Hybridization can also include self-hybridization to form intramolecular secondary structures (e.g. "hairpin" structures) such as those sensed by the SSCP analysis method. Even some forms of nuclease digestion such as digestion with restriction enzymes or RNAse H rely on hybridization of nucleic acid strands as part of the overall analysis process.

In carrying out the methods described it is to be understood that reference to particular buffers, media, reagents, cells, culture conditions, pH and the like are not intended to be limiting, but are to be read so as to include all related materials that one of ordinary skill in the art would recognize as being of interest or value in the particular context in which that discussion is presented. For example, it is often possible to substitute one buffer system or culture medium for another and still achieve similar, if not identical, results. Those of skill in the art will have sufficient knowledge of such systems and methodologies so as to be able, without undue experimentation, to make such substitutions as will optimally serve their purposes in using the methods and procedures disclosed herein.

### Examples

The present examples are provided for illustrative purposes only, and should not be construed as limiting the scope of the present invention as defined by the appended claims.

### Example 1

### Improvements of the Specific Amplification of Fosmid DNA from Cell Culture or the Mixture of Fosmid DNA and E.Coli DNA by Using Exonuclease mix (Exo I and Exo III)

### a) Multiply-Primed Rolling Circle Amplification with 2 µl of cell culture or the mixture of fosmid DNA and E.Coli DNA without the treatment of Exonuclease mix (Exo I and Exo III)

Amplification was carried out starting with 2 µl of cell culture of a randomly selected clone from a library of fosmid DNA in pCC1Fos vector (fosmid H1, overnight, LB medium with 12.5 µg chloramphenicol per ml, 40 kb insert DNA , from DOE Joint Genome Institute--JGI) or 2 µl of the mixture of the same fosmid DNA(purified by Qiagen kit) and E.Coli DNA(for example E.Coli DNA from USB, 10 ng/µl and JGI Fosmid DNA was purified by Qiagen kit, 0.1 ng/µl) in a 20 µl reaction volume containing 50 mM Tris-HCl, pH 8.25, 20 mM MgCl₂., 0.01% Tween-20, 75 mM KCl, 0.4mM dATP, 0.4 mM dTTP, 0.4 mM dCTP and 0.4 mM dGTP, 400 pmoles (800 ng) of random hexamer and 400 ng phi 29 DNA polymerase. The reaction mixture was incubated at 30° C for 5 hours to allow amplification of the DNA, and then incubated at 65° C for 10 minutes to inactivate the polymerase. Typical yield is 3-4 µg of DNA product as measured by fluorescence assay using Picogreen dye (Molecular Probes).

### b) Multiply-Primed Rolling Circle Amplification with 2 µl of cell culture or the mixture of fosmid DNA and E.Coli DNA with the treatment of Exonuclease mix (Exo I and Exo III)

The above standard amplification reaction was performed after the digestion of the JGI fosmid H1 cell culture or the mixture DNA of JGI fosmid H1 DNA and E.Coli DNA with Exonuclease mix (Exo I, 1 unit/µl, USB and Exo III, 10 unit /µl). The digestion was carried out at 37°C for 10 min, 30 min and 60 min respectively in a 20 µl reaction containing 66 mM Tris-HCl, pH 8.0, 6.6 mM MgCl₂, 5 mM DTT, and 0.05 mg/ml BSA. Then 2 µl of above reaction was transferred in a 20 µl reaction containing 50 mM Tris-HCl, pH 8.25, 20 mM MgCl₂., 0.01% Tween-20, 75 mM KCl, 0.4mM dATP, 0.4 mM dTTP, 0.4 mM dCTP and 0.4 mM dGTP, 400 pmoles (800 ng) of random hexamer and 400 ng phi 29 DNA polymerase. The reaction was incubated at 30° C for 5 hours to allow amplification of the DNA, and then incubated at 65° C for 10 minutes to inactivate the polymerase. Typical yield is 3-4 µg of DNA product as measured by fluorescence assay using Picogreen dye (Molecular Probes).

### c) Hind III digestion of Multiply-Primed Rolling Circle Amplification from 2 µl of cell culture or the mixture of fosmid DNA and E.Coli DNA with or without the treatment of Exonuclease mix (Exo I and Exo III)

Multiply-Primed Rolling Circle Amplification as described in detail in Example 1. After incubation 5 hrs at 30°C, 2 µl of each reaction mixture was digested with 15 units of HindIII for 3 hours at 37°C in a 20 µl reaction volume containing 10 mM Tris-HCl (pH 8.0), 7 mM MgCl₂, 60 mM NaCl and 2 µg bovine serum albumin. The products of the digestions along with the DNA marker were electrophoretically separated on a 0.9% agarose gel in 1x TBE buffer (89 mM Tris base, 89 mM boric acid, 2 mM EDTA, pH 8.3).

The data suggests that a 5 fold improvement of fosmid DNA specific amplification has been achieved from cell culture or the mixture of fosmid DNA and E.Coli DNA by using Exonuclease mix (Exo I and Exo III).

### Example 2

### Demonstration of Specific Degradation of Linear E. coli DNA versus Fosmid DNA by Real Time PCR

Real time PCR was carried out on an ABI 7900HT instrument with GEHC 16s rRNA primers for the detection of *E. coli* genomic DNA or JGI H1 primers for the detection of JGI fosmid H1 DNA

For Gelstar detection qPCR reactions the following primers were used at 1.2 µM final concentration with Gelstar at 1:25000 dilution in the final volume:

| | |
|---|---|
| Primers: | 16sF 5'-TTGACGTTACCCGCAGAAGAA-3' (SEQ ID NO.1) |
| | 16sR 5'-TGCGCTTTACGCCCAGTAAT-3' (SEQ ID NO. 2) |
| template: | bacterial genomic DNA used at 10-100000 copies per reaction |
| | |
| Primers: | JGI H1 F 5'-TCCCTACCGATTATGAGTTTG-3' (SEQ ID NO. 3) |
| | JGI H1 R 5'-CCCTTGGCCTAGGATACAAG-3' (SEQ ID NO. 4) |
| template: | JGI fosmid DNA used at 10-100000 copies per reaction |

Reactions were set up in a total volume of 25 µl which containing 1 x PCR buffer, 200 µM dNTPs, and 0.25 unit of Taq polymerase (Roche PCR kit). To these reactions either 5 µl of JGI cells culture treated with or without Exonuclease mix (Exo I and Exo III, see the detail information in the Example 1) or 5 µl of standard *E. coli* genomic DNA/ fosmid DNA was added per reaction.

### Cycling conditions:

| | |
|---|---|
| Stage 1: | 94°C 5 minutes |
| Stage 2 (50 repeats): | 94°C 30 seconds |
| | 48°C 30 seconds |
| | 72°C 60 seconds |
| Stage 3: | default denaturation |

The data demonstrate that greater than 99% E. Coli DNA was degraded whereas about 50% fosmid DNA were remained intact after Exonuclease mix (Exo I and Exo III) treatment with fosmid cell culture based on real time PCR.

### Example 3

### Significant improvement of BAC 89N6 DNA and fosmid DNA sequencing from glycerol stock by using Exonuclease mix (Exo I and Exo III)

BAC 89N6 clone which has a 92 Kb human DNA insert from chromosome 4 (a library of RPC 11 in the vector pBeloBAC 11) was treated with or without Exonuclease mix before being amplified by Multiply-Primed Rolling Circle Amplification as described in detail in Example 1. Then sequencing reactions were carried out using 5 pmoles of SP6 sequencing primer (5'-CGATTTAGGTGACACTATAG-3') (SEQ ID NO. 5) and 8 µl of DYEnamic ET terminator premix(GE Healthcare) and 2 µl of the amplified DNA. Reactions were cycled at normal temperatures (40 times at 95°C, 20 seconds, 50°C, 30 seconds and 60°C, 120 seconds). A randomly selected clone from a library of fosmid DNA in pCC1Fos vector (JGI fosmid H1, VTK 0529, 40 kb insert DNA) was treated with or without Exonuclease mix before being amplified by Multiply-Primed Rolling Circle Amplification as described in detail in Example 1. Then sequencing reactions were carried out using 5 pmoles of T7 sequencing primer (5'-TAATACGACTCACTATAGGG -3') (SEQ ID NO.6) and 8 µl of DYEnamic ET terminator premix and 2 µl of the amplified DNA. Reactions were cycled at normal temperatures (40 times at 95°C, 20 seconds, 50°C, 30 seconds and 60°C, 120 seconds). Samples were precipitated by ethanol, dissolved in 20 µl of 95% formamide and run on an ABI 3730 capillary sequencing instrument (Applied Biosystems Inc). The electropherogram obtained with the clone is shown in Figures 5 (BAC 89N6, without Exo mix treatment), in Figures 6 (BAC 89N6, with Exonuclease mix treatment), in Figures 7 (JGI fosmid H1, without Exonuclease mix treatment) and in Figures 8 (JGI fosmid H1, with Exonuclease mix treatment).

The data demonstrate significant improvement significant improvement of the sequencing read length of BAC DNA and fosmid DNA at least by 100base pairs from glycerol stock by using Exonuclease (Exo I and Exo III) mix.

### Example 4

### Improvement of Amplification of Fosmid DNA Obtained from Glycerol Stocks When Treated with Exonuclease Mix than Plasmid Safe ATP Dependent DNase

A randomly selected clone from a library of fosmid DNA in pCC1Fos(JGI fosmid H1, 40 kb insert of DNA, VTK 0529, glycerol stock) was treated with or without Exonuclease mix or Plasmid Safe ATP Dependent DNase (Epicentre) before being amplified by Multiply-Primed Rolling Circle Amplification as described in detail in Example 1.

After incubation 5 hrs at 30°C, 2 µl of each reaction mixture was digested with 15 units of HindIII for 3 hours at 37°C in a 20 µl reaction volume containing 10 mM Tris-HCl (pH 8.0), 7 mM MgCl₂, 60 mM NaCl and 2 µg bovine serum albumin. The products of the digestions along with the DNA marker were electrophoretically separated on a 0.9% agarose gel in 1x TBE buffer (89 mM Tris base, 89 mM boric acid, 2 mM EDTA, pH 8.3).

These data indicate that about a 4 fold better amplification of fosmid DNA was obtained from glycerol stocks treated with Exonuclease mix than Plasmid Safe ATP Dependent DNase

### Example 5

### Exonuclease Mix Enhances Specific Amplification of not only Fosmid DNA, but also BAC DNA from Glycerol Stocks using GenomiPhi v2 kit (GE Healthcare)

Two randomly selected clones from a library of BAC DNA (JGI BAC H1&C1, RPCI 11 clones, 175 kb insert of DNA, glycerol stock) and two randomly selected clones from a library of fosmid DNA in pCC1Fos (JGI fosmid H1&C1, 40 kb insert of DNA, VTK 0529, glycerol stock) along with BAC 89N6 glycerol stock were treated with or without Exonuclease mix before being amplified by Multiply-Primed Rolling Circle Amplification as described in detail in Example 1.

After incubation 5 hrs at room temperature, 2 µl of each reaction mixture was digested with 15 units of HindIII for 3 hours at 37°C in a 20 µl reaction volume containing 10 mM Tris-HCl (pH 8.0), 7 mM MgCl₂, 60 mM NaCl and 2 µg bovine serum albumin. The products of the digestions along with the DNA marker were electrophoretically separated on a 0.9% agarose gel in 1x TBE buffer (89 mM Tris base, 89 mM boric acid, 2 mM EDTA, pH 8.3).

The data demonstrate that the Exonuclease mix enhances not only specific fosmid DNA amplification, but also specific BAC DNA amplification from glycerol stocks using GenomiPhi v2 kit.

Although the present invention has been described above in terms of specific embodiments, many modification and variations of this invention can be made as will be obvious to those skilled in the art.

**Table 1-Representative Exonucleases**

| **Enzyme** | **Substrate** | **Directionality** | **Comments** | **Functionally Similar enzymes** |
|---|---|---|---|---|
| ***E.coli* ExoDNases** | | | | |
| ExoI | ss | 3'-5' | Highly Processive, Structure Solved | |
| Exo III | ds | 3'-5' | Strong AP endonuclease | |
| Exo VII | ss | 3'-5' 3'-5' | Oligonucleotide products, no metal required | |
| Exo VIII (Rec E) | ds | 5'-3' | High processivity | |
| Exo IX | ss | 3'-5' | Also 3' phosphodiesterase at 3' incised AP sites | |
| Exo X | ss & ds | 3'-5' | | |
| DNA pol I 5'-3' exo | ss | 5'-3' | (Formerly ExoVI) | FenI, T5-D15, hsDNaseIV Tth pol 5'-3'exo scRad27, |
| DNA pol I 3'-5' exo | ss & mispaired 3' termini | 3'-5' | Proofreading exo (formerly ExoII) | Many proofreading 3'-5' polymerase associated exos |
| DNA pol II 3'-5' exo | ss | 3'-5' | Proofreading exo | |
| DNA pol III 3'-5' exo - ε subunit (DnaQ, mutD) | ss & mispaired 3' termini | 3'-5' | Proofreading exo | |
| RecBCD | ss & ds | 5'-3', 3'-5' | ATP dependent, helicase activity, Oligonucleotide products, (formerly ExoV) | M.Luteus ExoV |
| Rec J | ss | 5'-3' | | |
| SbcCD | ds | | ATP and Mn⁺⁺ dependent, processive | sc -Mre11 sp-RAD32 |
| **Other ExoDNases** | | | | |
| Lambda Exonuclease | ds | 5'-3' | Highly processsive, structure solved | |
| Bal 31 | ds | | From *Alteromonus espejina* has strong endonuclease activity on ssDNA | |
| P2 - Old Exonuclease | ds | 5'-3' | | |
| T7 gene 6 | ds | 5'-3' | | |
| *S. cerevisiae* ExoI | ds | 5'-3' | Mismatch repair | spExoI |
| Human Rad9 | | 3'-5' | Implicated in cell cycle regulation | spRad9 |
| | | | | |
| ***E.coli* ExoRNases** | | | | |
| RNase II | | 3'-5' | | scRrp44p |
| RNase D | | 3'-5' | | scRrp6p |
| RNase BN | | 3'-5' | | |
| RNase T | | 3'-5' | | |
| RNase R | | 3'-5' | | |
| Oligoribonuclease | | 3'-5' | | |
| RNase PH | | 3'-5' | Phosphorylase (NDP products) | scRrp41 p |
| PNPase | | 3'-5' | Phosphorylase (NDP products) | |
| **Other ExoRNases** | | | | |
| S. cerevisiae Xrn I | | 5'-3' | | |
| S. cerevisiae Rat I | | 5'-3' | | |
| | | | | |
| **Phosphodiesterases** | | | | |
| Spleen Phosphodiesterase | ss | 5'-3' | | |
| Venom Phosphodiesterase | ss, ds | 3'-5' | Can nick superhelical DNA | |

## Claims

1. A method for amplifying circular DNA, comprising:
a) lysing a host cell carrying a form of circular DNA of interest to form a lysate,
b) digesting this lysate with two or more purified exonuclease enzymes in a manner that does not digest the circular DNA,
c) optionally inactivating the two or more exonuclease enzymes,
d) adding multiple single stranded oligonucleotide primers, a DNA polymerase and deoxynucleoside triphosphates, and;
e) incubating said mixture under conditions wherein said amplification target binds to more than one of said primers to promote replication of said amplification target by extension of primers to form multiple amplified DNA products, wherein said incubating step is performed isothermally.

2. The method of claim 1, wherein said cell is a bacterial cell.

3. The method of claim 1, wherein said cell is a eukaryotic cell.

4. The method of claim 3, wherein said eukaryotic cell is a yeast cell

5. The method of claim 1, wherein the circular DNA is an episome.

6. The method of claim 5, wherein said episome is a plasmid.

7. The method of claim 5, wherein said episome is a fosmid.

8. The method of claim 5, wherein said episome is a bacterial artificial chromosome.

9. The method of claim 3, wherein the circular DNA is, or is, derived from mitochondrial DNA.

10. The method of claim 5, wherein the said episome has a DNA sequence insert.

11. The method of claim 1, wherein the amplification method is Rolling Circle Amplification.

12. The method of claim 1 in which nuclease resistant oligonucleotides and dNTPs are used and the exonuclease enzymes comprise *E.coli* Exonuclease I and III.

## Patentansprüche

1. Verfahren zum Amplifizieren von ringförmiger DNA umfassend:
a) Lysieren einer Wirtszelle enthaltend eine Form von ringförmiger DNA von Interesse, um ein Lysat zu bilden,
b) Verdauen dieses Lysats mit einem oder mehreren Exonuklease-enzymen auf eine Art und Weise wobei die ringförmige DNA nicht verdaut wird,
c) gegebenenfalls Inaktivieren der zwei oder mehreren Exonuklease-enzyme,
d) Zugeben einer Vielzahl von einzelsträngigen Oligonukleotidprimern, einer DNA-Polymerase und Desoxynukleosidtriphosphaten, und
e) Inkubieren der Mischung unter Bedingungen wobei das Amplifikationstarget an mehr als einen der Primer bindet, um die Replikation des Amplifikationstargets durch eine Verlängerung von Primern zu begünstigen, so dass eine Vielzahl von amplifizierten DNA-Produkten gebildet wird, wobei der Inkubierschritt isothermisch durchgeführt wird.

2. Verfahren nach Anspruch 1, wobei die Zelle eine bakterielle Zelle ist.

3. Verfahren nach Anspruch 1, wobei die Zelle eine eukaryotische Zelle ist.

4. Verfahren nach Anspruch 3, wobei die eukaryotische Zelle eine Hefezelle ist.

5. Verfahren nach Anspruch 1, wobei die ringförmige DNA ein Episom ist.

6. Verfahren nach Anspruch 5, wobei das Episom ein Plasmid ist.

7. Verfahren nach Anspruch 5, wobei das Episom ein Fosmid ist.

8. Verfahren nach Anspruch 5, wobei das Episom ein bakterielles künstliches Chromosom ist.

9. Verfahren nach Anspruch 3, wobei die ringförmige DNA mitochondriale DNA ist oder davon abgeleitet ist.

10. Verfahren nach Anspruch 5, wobei das Episom ein DNA-Sequenz-Insert aufweist.

11. Verfahren nach Anspruch 1, wobei das Amplifikationsverfahren eine Rolling-Circle-Amplifikation ist.

12. Verfahren nach Anspruch 1 worin Nuklease-resistente Oligonukleotide und dNTPs verwendet werden und die Exonukleaseenzyme Exonuklease I und III von *E. coli* umfassen.

## Revendications

1. Procédé d'amplification d'un ADN circulaire, comprenant :
a) la lyse d'une cellule hôte portant une forme d'ADN circulaire d'intérêt pour former un lysat,
b) la digestion de ce lysat avec deux ou plus de deux enzymes exonucléases purifiées de telle manière que l'ADN circulaire ne soit pas digéré,
c) facultativement, l'inactivation des deux ou plus de deux enzymes exonucléases,
d) l'ajout de multiples amorces oligonucléotidiques monocaténaires, d'une ADN polymérase et de désoxynucléoside-triphosphates, et ;
e) l'incubation dudit mélange dans des conditions dans lesquelles ladite cible d'amplification se lie à plus d'une desdites amorces pour favoriser la réplication de ladite cible d'amplification par l'extension des amorces pour former de multiples produits d'ADN amplifiés, dans laquelle ladite étape d'incubation est réalisée de manière isothermique.

2. Procédé selon la revendication 1, dans lequel ladite cellule est une cellule bactérienne.

3. Procédé selon la revendication 1, dans lequel ladite cellule est une cellule eucaryote.

4. Procédé selon la revendication 3, dans lequel ladite cellule eucaryote est une cellule de levure.

5. Procédé selon la revendication 1, dans lequel l'ADN circulaire est un épisome.

6. Procédé selon la revendication 5, dans lequel ledit épisome est un plasmide.

7. Procédé selon la revendication 5, dans lequel ledit épisome est un fosmide.

8. Procédé selon la revendication 5, dans lequel ledit épisome est un chromosome artificiel bactérien.

9. Procédé selon la revendication 3, dans lequel l'ADN circulaire est un ADN mitochondrial ou dérive d'un ADN mitochondrial.

10. Procédé selon la revendication 5, dans lequel ledit épisome comporte un insert de séquence d'ADN.

11. Procédé selon la revendication 1, dans lequel le procédé d'amplification est une amplification en cercle roulant.

12. Procédé selon la revendication 1, dans lequel des oligonucléotides et des dNTP résistant aux nucléases sont utilisées et les enzymes exonucléases comprennent les exonucléases I et III d'E. *coli.*
